# EUROPÄISCHE PATENTSCHRIFT

(11) **EP 0 515 602 B1**
(45) Veröffentlichungstag und Bekanntmachung des Hinweises auf die Patenterteilung: **10.01.1996**
(21) Anmeldenummer: 91920762.1
(22) Anmeldetag: 30.11.1991
(51) Int. Cl.: C09C 1/00

(54) **OBERFLÄCHENMODIFIZIERTE PLÄTTCHENFÖRMIGE SUBSTRATE**
SURFACE-MODIFIED FLAKE-LIKE SUBSTRATES
SUBSTRATS LAMELLAIRES MODIFIES SUPERFICIELLEMENT

(30) Priorität: 12.12.1990 DE 4039593
(43) Veröffentlichungstag der Anmeldung: 02.12.1992
(73) Patentinhaber: MERCK PATENT GmbH, D-64271 Darmstadt (DE)
(72) Erfinder: HERGET, Gerhard, D-6105 Ober-Ramstadt (DE)
(86) Internationale Anmeldenummer: EP9102270
(87) Internationale Veröffentlichungsnummer: WO9210545

(56) Entgegenhaltungen:
- GB-A- 1 112 362
- US-A- 3 905 936
- US-A- 4 327 017
- US-A- 4 648 908
- CHEMICAL ABSTRACTS, vol. 107, no. 16, 19. Oktober 1987, Columbus, Ohio, US; abstract no. 135963Q, Seite 92; Spalte R; & CS-B-236728 (NEDOROST, M. ET AL.) 15.11.1986 see abstract

## Beschreibung

Die Erfindung betrifft oberflächenmodifizierte plättchenförmige Substrate sowie deren Herstellungsverfahren und deren Verwendung.

Plättchenförmige Substrate wie z.B. Metalle, Metalloxide oder mit Metalloxiden beschichtete plättchenförmige Materialien finden z.B. als Effektpigmente in vielen technischen Bereichen Verwendung.

Die immer größer werdende Einsetzbarkeit dieser Pigmente in unterschiedlichen technischen Bereichen erfordert zunehmend die Entwicklung oberflächenbelegter und modifizierter Substrate um letztlich die Verträglichkeit der Substrate mit weiteren Komponenten innerhalb technischer Zusammensetzungen zu gewährleisten. Die Pigmente finden sowohl zur Pigmentierung von Lacken, Pulverlacken, Farben, Druckfarben, Kunststoffen und dergleichen als auch in kosmetische Zubereitungen Verwendung.

Für das große Einsatzgebiet der Pigmente benötigt man Pigmente hervorragender photochemischer Stabilität sowie guter Haltbarkeit und der Fähigkeit Lackierungen und Überzügen einen hohen Glanz zu verleihen.

Es ist an sich bekannt, Pigmente mit organischen Komponenten zu beschichten, Verfahren zur Herstellung solcher Pigmente sind z.B. beschrieben in den DE-OS 37 12 289, EP-PS 0090259 und DE-OS 36 27 329.

Aus der DE-OS 22 15 191 ist bekannt, glimmerhaltige Pigmente mit einem Überzug aus Methacrylatochrom(III)chlorid zu überziehen, was zu einer Verbesserung der Lichtbeständigkeit führt. In der DE-PS 26 03 211 wird ein Verfahren zur Beschichtung von feinteiligen Pigmenten mit einem Polymerharz zur Verbesserung der Dispergierbarkeit beschrieben.

In der U.S. 3,905,936 werden u.a. Perlglanzpigmente mit einem Dialkoxyaluminiummethacrylat oder -acrylat in Gegenwart eines Lösungsmittels nachbeschichtet. Aus der U.S. 4,327,017 sind Perlglanzpigmente bekannt, die mit einem Polyaluminiumsalz und einem Harz, z.B. Abietinsäure bzw. deren Salze nachbeschichtet werden.

In der US-A-4 648 908 werden oberflächenmodifizierte Perlglanzpigmente beschrieben, die mit einer Aluminiumseite, die mindestens 20 gew% Wasser enthält, beschichtet sind.

In der GB-A-1 112 362 sind mit Aluminiumtricarboxylaten beschichtete Glimmerplätchen beschrieben

In der CS-B-236 728 wird ein Verfahren zur Hydrophobie ring von Perlglanzpigmenten beschrieben, wobei die Perlglanzpigmente in wäßriger Lösung mit einem Aluminiumstearat, - palmitat, - phtalat oder Naphtenat beschichtet werden.

Die Oberflächenbelegung der Substrate beinhaltet dabei stets, daß das Pigment in einer Suspension vorgelegt wird, mit dem Modifiziermittel versetzt und dieses auf das Pigment aufgefällt wird. Anschließend wird getrocknet und das Pigment in eine anwendungsgerechte Form gebracht.

Aufgabe der vorliegenden Erfindung war es, neue Pigmente zu finden, die kompatibel für die verschiedensten technischen Formulierungen sind. Eine weitere Aufgabe bestand darin, ein Herstellungsverfahren zu finden, das ein direktes Belegen des Modifiziermittels auf die Pigmentteilchen erlaubt, ohne daß zusätzliche Verfahrensschritte notwendig sind.

Es wurde nun überraschend gefunden, daß neue oberflächenmodifizierte Substrate durch Behandlung von Basissubstraten mit organischen Aluminiumverbindungen entstehen, wobei dem Modifiziermittel auch organische Siliciumverbindungen zugesetzt werden können. Effektpigmente sind bisher nicht in dieser Weise behandelt worden. Ebenso überraschend ist, daß die Pigmente nach dem erfindungsgemäßen Verfahren in ihren optischen Eigenschaften den bisher bekannten oberflächenbelegten Pigmenten nicht nachstehen. So kann der Anteil des Modifiziermittels in den Pigmentzubereitungen bis zu etwa 25 % betragen, ohne daß nach der Verarbeitung Beeinträchtigungen der optischen Eigenschaften zu vermerken sind. Andererseits werden aber auch schon qualitativ einwandfreie pulverförmige Effektpigmente erhalten, wenn der Gehalt des Modifiziermittels nur etwa % beträgt. Bevorzugt wird jedoch der Bereich von 1 bis 10 % und insbesondere der Bereich von 1,5 bis 6 Die Prozentzahlen sind dabei Gewichtsprozent des Modifiziermittels, bezogen auf die gemäß der Erfindung hergestellten Pigmentzubereitungen.

Gegenstand der Erfindung sind somit oberflächenmodifizierte plättchenförmige Substrate, dadurch gekennzeichnet, daß sie mit einem Modifizierungsreagenz bestehend aus Aluminiumalkoholaten oder -acetylacetylaten oder einem Gemisch aus Aluminiumalkoholaten bzw. -acetylacetylaten und Siliziumverbindungen sowie Partikeln, insbesondere Farbpigmente oder magnetische Partikel, beschichtet sind, wobei der Anteil an Modifizierungsreagenz bezogen auf das Gesamtpigment bis zu 25 Gew.% beträgt, mit der Maßgabe, daß bei den Partikeln Aluminiumalkoholate bzw. -acetylacetylate ausgeschlossen sind.

Gegenstand der Erfindung ist auch ein Verfahren zur Herstellung oberflächenmodifizierter plättchenförmiger Substrate, dadurch gekennzeichnet, daß man in einem Mischgefäß plättchenförmige Substrate mit dem Modifizierungsmittel, bestehend aus Aluminiumalkoholaten oder -acetylacetylaten oder einem Gemisch aus Aluminiumalkoholaten bzw. Acetyacetylaten und Siliziumverbindungen sowie Partikeln, insbesondere Farbpigmente oder magnetische Partikel in Abwesenheit eines Lösungsjmittels in Berührung bringt, anschließend das erhaltene Produkt trocknet und gegebenenfalls glüht.

Gegenstand der Erfindung ist weiterhin der Einsatz der erfindungsgemäßen Substrate in Farben, Druckfarben, Lacken, Pulverlacken, Kunststoffen, kosmetischen Präparaten und in Bronzierverfahren sowie als Ausgangsmaterial zur Herstellung weiterer modifizierter Produkte.

Alle bekannten plättchenförmigen Metalle, Metalloxide, Glimmerpigmente und sonstigen plättchenförmigen Substrate können nach dem erfindungsgemäßen Verfahren belegt werden. Besonders bevorzugt werden jedoch Perlglanzpigmente auf Basis von mit Metalloxiden, insbesondere Titanoxid und/oder Eisenoxid, beschichteten Glimmerschuppen eingesetzt. Alle diese Pigmente sind bekannt und sind sowohl nach bekannten Methoden herstellbar als auch im Handel erhältlich z.B. unter dem Warenzeichen Iriodin von der Firma E. Merck, Darmstadt.

Das erfindungsgemäße Verfahren läßt sich wie folgt beschreiben: Die genannten Ausgangssubstrate werden in einem Mischgefäß, z.B. in einer Lädige-Trommel, einem Konus- oder Taumelmischer, vorgelegt und unter intensivem Mischen mit dem Modifiziermittel versetzt, so daß die Pigmente möglichst homogen mit dem Modifiziermittel ummantelt werden. Das Modifiziermittel wird dabei rein zugesetzt. Das plättchenförmige Substrat wird somit mit dem Modizifiermittel benetzt. Die Beschichtung selbst geschieht somit allein durch Mischen der Komponenten.

Als Modifiziermittel werden Aluminiumalkoholate insbesondere Alkoholate mit den Resten Ethyloxy, n-Propyloxy, iso-Propyloxy, n-Butyloxy, iso-Butyloxy, t-Butyloxy, n-Hexyloxy, n-Octyloxy, 2-Ethylhexyloxy und n-Decyloxy, sowie Acetylacetylate verwendet. Es können auch Gemische aus zwei oder drei Alkoholaten bzw. Acetylacetylaten verwendet werden. Besonders geeignet sind hierbei Gemische aus Aluminiumisopropylat/-isobutylat und Aluminiumisopropylat/-2-ethylhexylat. Weiterhin werden als Modifiziermittel Gemische aus Aluminiumalkoholaten, -acetylacetylaten und organischen Siliciumverbindungen insbesondere Siliciumtetrachlorid und Tetraethoxysilan eingesetzt.

Farblich modifizierte oberflächenbelegte Effektpigmente entstehen, indem man dem Modifiziermittel einen Farbstoff oder ein Farbpigment wie z.B. Kupferphthalocyanin , Ruß, oder Eisenoxid beimischt.

Solche Farbmittel sind beschrieben etwa in Lückert, Pigment und Füllstofftabellen, 4. Aufl. 1989 Herbst, Hunger, Industrielle Organische Chemie, VCH 1987 oder Industrielle Anorganische Chemie, VCH 1987.

Mikroskopische Untersuchungen belegen, daß die farbgebende Komponente gleichmäßig auf das Effektpigment aufgezogen ist und keine Nebenfällung vorliegt.

Das Pigmentpulver ist weiterhin lösemittelecht und abrasionsfest.

Gegenstand der Erfindung ist damit auch, daß man dem Modifiziermittel zusätzlich Partikel, insbesondere Farbpigmente oder magnetische Partikel zusetzt.

Unter dem gezielten Einfluß von Feuchtigkeit und/oder Hitze verankert sich das Modifiziermittel auf der Pigmentoberfläche. Anschließend wird das Pigment aus dem Mischer ausgetragen und gesiebt. Nach dem Trocknungsprozeß liegt die Pigmentzubereitung als frei fließendes Pulver vor.

In dem erfindungsgemäßen Verfahren wird somit der Zwischenschritt der Aufschlämmung, Fällung und Filtration umgangen. Gerätekosten sowie Abwässerprobleme können durch das erfindungsgemäße Verfahren deutlich reduziert werden.

Die Herstellung des Pigments kann damit beendet sein. Um die Oberfläche gezielt einzustellen, kann auch ein Trockengang oder Glühgang bei definierter Temperatur vorzugsweise 100-600 °C angeschlossen werden. Die Oberfläche zeichnet sich durch einen hohen Anteil an basischen Zentren aus und bildet somit eine gute Ausgangsbasis für nachgeschaltete organische Belegungen.

Behandelt man das nach dem erfindungsgemäßen Verfahren hergestellte fertige Pigment mit einem Dispergiermittel, insbesondere mit modifizierten Polyacrylaten, so erhält man Pigmente mit einem ausgezeichneten Redispergierverhalten. Solche Polyacrylate sind aufgeführt z.B. in Karsten, Lackrohstofftabellen, 8. Auflage 1987.

Gegenstand der Erfindung ist somit auch die Verwendung der erfindungsgemäßen oberflächenbelegten Substrate als Ausgangsmaterial für weitere modifizierte Produkte, z.B. führt die Nachbehandlung mit Myristinsäure zu einem Pulver mit ausgezeichnetem Rieselverhalten.

Die erfindungsgemäßen Pigmente zeichnen sich durch eine Reihe von Vorteilen gegenüber den nach herkömmlichen Verfahren hergestellten oberflächenbelegten Pigmenten aus. Beispielsweise zeigen die nach dem erfindungsgemäßen Verfahren hergestellten Pigmente ein verbessertes Dispergier- und Redispergierverhalten. Aufgrund der geringen Kohäsion der Pigmentteilchen ist dieses freifließende Material hervorragend für das Druck- und Bronzierverfahren geeignet. Versuche haben gezeigt, daß nach dem erfindungsgemäßen Verfahren behandelte Eisenoxidpigmente einen größeren Schutz gegen Abrieb aufweisen, und daß die erfindungsgemäßen Pigmente ein verbessertes skin-feeling besitzen.

Zur Erläuterung der Erfindung dienen die folgenden Beispiele.

### Beispiel 1

15 g Kupfer-phthalocyanin werden in 85 g Isopropanol dispergiert und unter Feuchtigkeitsauschluß mit 12,0 g Aluminium-isopropylat versetzt. 24 g dieser Farbmitteldispersion werden zu 194 g Ti0₂/Fe₂0₃-Glimmer (Teilchengröße 10-50 µm) in 50 ml Isopropanol zugegeben. Danach wird intensiv gerührt und das Lösungsmittel bei 70 °C abgedampft.

Das so erhaltene farblich modifizierte Pigment zeigt einen grünlichen Goldeffekt.

### Beispiel 2

Das aus Beispiel 8 erhaltene modifizierte Pigment wird unter starkem Rühren mit einer Mischung aus 2 g Myristinsäure in 18 g Aceton versetzt.

Nach Entfernung des Lösungsmittels erhält man ein leicht rieselndes Pulver, das für das Bronzierverfahren geeignet ist.

## Patentansprüche

1. Oberflächenmodifizierte plättchenförmige Substrate, dadurch gekennzeichnet, daß sie mit einem Modifizierungsreagenz bestehend aus Aluminiumalkoholaten oder - acetylacetylaten oder einem Gemisch aus Aluminiumalkoholaten bzw. -acetylacetylaten und Siliziumverbindungen sowie Partikeln, insbesondere Farbpigmente oder magnetische Partikel, beschichtet sind, wobei der Anteil an Modifizierungsreagenz bezogen auf das Gesamtpigment bis zu 25 Gew.% beträgt, mit der Maßgabe, daß bei den Partikeln Aluminiumalkoholate ausgeschlossen sind.

2. Oberflächenmodifizierte plättchenförmige Substrate nach Anspruch 1, dadurch gekennzeichnet, daß das Aluminiumalkoholat Aluminiumethylat, -propylat oder -butylat oder ein Gemisch aus Aluminiumisopropylat / -isobutylat oder Aluminiumisopropylat / -2-ethylhexylat ist.

3. Oberflächenmodifizierte plättchenförmige Substrate nach Anspruch 1 oder 2, dadurch gekennzeichnet, daß die Siliziumverbindung Siliziumtetrachlorid oder Tetraethoxysilan ist.

4. Oberflächenmodifizierte plättchenförmige Substrate nach einem der Ansprüche 1 bis 3, dadurch gekennzeichnet, daß das plättchenförmige Substrat ein Perlglanzpigment ist.

5. Verfahren zur Herstellung oberflächenmodifiherfer plättchenförmiger Substrate nach Anspruch 1, dadurch gekennzeichnet, daß man in einem Mischgefäß plättchenförrnige Substrate mit dem Modifiherungsmittel, bestehend aus Aluminiumalkoholaten oder -acetylacetylaten oder einem Gemisch aus Aluminiumalkoholaten bzw. Acetyacetylaten und Siliziumverbindungen sowie Partikeln, insbesondere Farbpigmente oder magnetische Partikel in Abwesenheit eines Lösungsmittels in Berührung bringt, anschließend das erhaltene Produkt trocknet und gegebenenfalls glüht.

6. Verwendung der Substrate nach Anspruch 1 als Ausgangsmaterial zur Herstellung weiterer modifizierter Produkte.

7. Verwendung der Substrate nach Anspruch 1 in Farben, Druckfarben, Lacken, Pulverlacken, Kunststoffen, kosmetischen Präparaten sowie im Bronzierverfahren.

## Claims

1. Surface-modified plateletlike substrates, characterized in that they have been coated with a modifying reagent consisting of aluminium alcoholates or acetylacetylates or a mixture of aluminium alcoholates or acetylacetylates and silicon compounds and also particles, in particular colour pigments or magnetic particles, the proportion of modifying reagent based on the total pigment being up to 25% by weight, with the proviso that the particles are not aluminium alcoholates.

2. Surface-modified plateletlike substrates according to Claim 1, characterized in that the aluminium alcoholate is aluminium methoxide, propoxide or butoxide or a mixture of aluminium isopropoxide, isobutoxide or aluminium isopropoxide/2-ethylhexoxide.

3. Surface-modified plateletlike substrates according to Claim 1 or 2, characterized in that the silicon compound is silicon tetrachloride or tetraethoxysilane.

4. Surface-modified plateletlike substrates according to any one of Claims 1 to 3, characterized in that the plateletlike substrate is a pearl lustre pigment.

5. Process for producing surface-modified plateletlike substrates according to Claim 1, characterized in that plateletlike substrates are brought into contact with the modifying agent consisting of aluminium alcoholates or acetylacetylates or a mixture of aluminium alcoholates or acetylacetylates and silicon compounds and also particles, in particular colour pigments or magnetic particles, in a mixing vessel in the absence of a solvent, the product obtained is then dried and optionally calcined.

6. Use of the substrates according to Claim 1 as starting material for the preparation of further modified products.

7. Use of the substrates according to Claim 1 in varnishes, printing inks, paints, powder coatings, plastics, cosmetic preparations and in the bronzing process.

## Revendications

1. Substrats lamellaires modifiés superficiellement, caractérisés en ce qu'ils sont revêtus d'un réactif de modification constitué d'alcoolates ou d'acétylacétylates d'aluminium ou d'un mélange d'alcoolates ou d'acétylacétylates d'aluminium et de composés du silicium, ainsi que de particules, en particulier des pigments colorés ou des particules magnétiques, la part du réactif de modification par rapport à l'ensemble des pigments valant jusqu'à 25 % en poids, avec la condition que, pour les particules, des alcoolates d'aluminium soient exclus.

2. Substrats lamellaires modifiés superficiellement selon la revendication 1, caractérisés en ce que l'alcoolate d'aluminium est un éthylate, propylate ou butylate d'aluminium ou un mélange d'isopropylate/isobutylate d'aluminium ou d'isopropylate/2-éthylhexylate d'aluminium.

3. Substrats lamellaires modifiés superficiellement selon la revendication 1 ou 2, caractérisés en ce que le composé de silicium est du tétrachlorure de silicium ou du tétraéthoxysilane.

4. Substrats lamellaires modifiés superficiellement selon l'une des revendications 1 à 3, caractérisés en ce que le substrat lamellaire est un pigment nacré.

5. Procédé de fabrication de substrats lamellaires modifiés superficiellement selon la revendication 1, caractérisé en ce qu'on met sous agitation dans un récipient de mélange, en l'absence d'un solvant, des substrats lamellaires avec l'agent de modification, constitué d'alcoolates ou d'acétylacétylates d'aluminium ou d'un mélange d'alcoolates ou d'acétylacérylates d'aluminium et de composés de silicium, ainsi que de particules, en particulier des pigments colorés ou des particules magnétiques, puis l'on sèche le produit obtenu et on le cuit le cas échéant.

6. Utilisation des substrats selon la revendication 1 comme matériau de base pour la fabrication d'autres produits modifiés.

7. Utilisation des substrats selon la revendication 1 dans des peintures, des peintures d'impression, des laques, des laques en poudre, des matières synthétiques, des préparations cosmétiques, ainsi que dans des procédés de bronzage.
